(19) Europäisches Patentamt
European Patent Office
Office européen des brevets

(11) **EP 3 212 802 B1**

(12) **FASCICULE DE BREVET EUROPEEN**

(45) Date de publication et mention
de la délivrance du brevet:
**27.11.2019 Bulletin 2019/48**

(21) Numéro de dépôt: **15798513.6**

(22) Date de dépôt: **28.10.2015**

(51) Int Cl.:
***C12Q 1/04*** *(2006.01)*

(86) Numéro de dépôt international:
**PCT/FR2015/052902**

(87) Numéro de publication internationale:
**WO 2016/066957 (06.05.2016 Gazette 2016/18)**

(54) **PROCÉDÉ DE DÉTECTION D'UNE CONTAMINATION À LA MÉRULE**

VERFAHREN ZUR ERKENNUNG VON HAUSSCHWAMMBEFALL

METHOD FOR DETECTING DRY ROT FUNGUS CONTAMINATION

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **31.10.2014 FR 1460498**

(43) Date de publication de la demande:
**06.09.2017 Bulletin 2017/36**

(73) Titulaire: **Centre Scientifique Et Technique Du
Batiment
(CSTB)
77420 Champs sur Marne (FR)**

(72) Inventeurs:
• **MOULARAT, Stéphane
F-77200 Torcy (FR)**
• **ROBINE, Enric
F-77600 Conches-sur-Gondoire (FR)**

(74) Mandataire: **Plasseraud IP
66, rue de la Chaussée d'Antin
75440 Paris Cedex 09 (FR)**

(56) Documents cités:
**EP-A1- 2 708 604      FR-A1- 2 913 501
GB-A- 2 401 174**

• **EWEN ET AL: "Identification by gas
chromatography-mass spectrometry of the
volatile organic compounds emitted from the
wood-rotting fungi Serpula lacrymans and
Coniophora puteana, and from Pinus sylvestris
timber", MYCOLOGICAL RESEARCH, ELSEVIER,
GB, vol. 108, no. 7, 1 juillet 2004 (2004-07-01),
pages 806-814, XP022443191, ISSN: 0953-7562,
DOI: 10.1017/S095375620400022X**
• **HAMILTON S ET AL: "Detection of Serpula
lacrymans infestation with a polypyrrole sensor
array", SENSORS AND ACTUATORS B:
CHEMICAL: INTERNATIONAL JOURNAL
DEVOTED TO RESEARCH AND DEVELOPMENT
OF PHYSICAL AND CHEMICAL TRANSDUCERS,
ELSEVIER S.A, CH, vol. 113, no. 2, 27 février 2006
(2006-02-27), pages 989-997, XP027971367, ISSN:
0925-4005 [extrait le 2006-02-27]**
• **KUSKE M ET AL: "Microbial volatile organic
compounds as indicators of fungi. Can an
electronic nose detect fungi in indoor
environments?", BUILDING AND
ENVIRONMENT, PERGAMON PRESS, OXFORD,
GB, vol. 40, no. 6, 1 juin 2005 (2005-06-01), pages
824-831, XP027748675, ISSN: 0360-1323 [extrait
le 2005-06-01]**

**Description**

La présente invention concerne un procédé de détection d'une contamination à la mérule dans des environnements intérieurs.

La mérule (*Serpula lacrymans*) est un champignon lignivore qui s'attaque aux bois, notamment le bois d'œuvre servant de structure dans de nombreux bâtiments. Dans les conditions favorables à sa croissance, la colonisation des produits par ce champignon peut entraîner des dégâts considérables en altérant les propriétés mécaniques du bois. En Europe du Nord, la mérule est responsable de 70 % des dégradations à l'intérieur des bâtiments, ce qui amène à considérer la mérule comme l'agent le plus nuisible dans un bâtiment.

Outre son implication dans la dégradation des produits, le rôle sensibilisant de ce champignon a été confirmé, depuis plusieurs années, chez des sujets atopiques et/ou asthmatiques par des tests de provocation bronchique.

Au vu de l'ampleur de cette problématique, les pouvoirs publics tendent à rendre obligatoire non seulement la détection de contamination à la mérule, mais aussi la déclaration des cas de mérule en mairie.

Aujourd'hui, une contamination à la mérule est détectée le plus souvent visuellement, lorsque le bois attaqué présente des stades avancés de dégradation. Une telle contamination est contrecarrée par l'élimination et le remplacement des pièces de bois infestées et le recours à des traitements essentiellement chimiques. De fait, il n'existe pas, à ce jour, de moyens de détection précoce, notamment lorsqu'il n'existe encore aucun signe visible de contamination, qui permettraient la mise en œuvre de mesures préventives.

Basée sur des travaux antérieurs qui ont abouti à l'élaboration d'un outil de détection de contaminations fongiques et notamment de contaminations cachées ou récentes (FR 2913501), une approche innovante est ici employée pour détecter une contamination à *Serpula lacrymans* dans les environnements intérieurs. Un objet de la présente invention est donc de pallier à tout ou partie des inconvénients cités ci-dessus.

*Serpula lacrymans,* comme tous les champignons, émet dès le début de son développement des molécules volatiles (Composés Organiques Volatils) issues soit de leur métabolisme, soit de la dégradation du matériau (ou substrat) sur lequel il se développe par les enzymes ou les acides qu'il produit. Contrairement aux spores, ces composés se dispersent dans l'environnement sans être retenus par les supports. Par conséquent la détection de certains de ces composés qui sont spécifiques de *Serpula lacrymans,* permet d'une part l'identification d'une contamination dès le début du développement de *Serpula lacrymans* et d'autre part la détection des contaminations « masquées » pour lesquelles il n'y a aucun signe de contamination visibles.

La Société Demanderesse a eu le mérite d'identifier les COV émit par *Serpula lacrymans* lors de son développement. Cependant, la simple détection de la présence de ces COV conduit à un nombre important de faux-négatifs ou de faux-positifs et ne permet donc pas conclure sur une contamination à *Serpula lacrymans* de façon suffisamment précise. En particulier, le développement de *Serpula lacrymans* s'accompagne fréquemment d'un développements d'ascomycètes alors que la réciproque est fausse. Il est donc important de pouvoir disposer d'une méthode de détection spécifique de contamination à *Serpula lacrymans* pour éviter les faux-positifs.

La Société Demanderesse a maintenant trouvé que, parmi les COV émis par *Serpula lacrymans* lors de son développement, certains COV ne sont présents dans les environnements intérieurs qu'en présence de contamination à *Serpula lacrymans.* Ces COV sont donc spécifiques au développement de *Serpula lacrymans.* Au contraire, d'autres COV émis par *Serpula lacrymans* lors de son développement peuvent être présents dans des environnements intérieurs ne présentant pas de contamination à *Serpula lacrymans.* Ces COV ne sont donc pas spécifiques au développement de *Serpula lacrymans* et peuvent avoir d'autres origines, notamment la présence de certains matériaux de construction ou encore d'autres contaminations biologiques telles que des contaminations à d'autres espèces de champignons ou bactériennes. Enfin, la Société Demanderesse a trouvé que certains des COV identifiés comme spécifiques au développement de *Serpula lacrymans* ne sont émis que par certaines souches de *Serpula lacrymans.* Par conséquent la Société Demanderesse a déterminé trois catégories distinctes de COV émis par *Serpula lacrymans* lors de son développement :

(1) les COV émis par *Serpula lacrymans* quelle que soit la souche de *Serpula lacrymans* et qui n'ont pas d'autres origines ;
(2) les COV émis par *Serpula lacrymans* quelle que soit la souche de *Serpula lacrymans,* mais qui peuvent avoir d'autres origines ;
(3) les COV émis seulement et spécifiquement par certaines souches de *Serpula lacrymans* et qui n'ont pas d'autres origines ;

C'est ainsi que la Société Demanderesse a eu le mérite, après des travaux de recherche approfondis, de développer un procédé de détection d'une contamination à *Serpula lacrymans* dans un environnement intérieur qui permette la détection d'une telle contamination même en l'absence de signes de contaminations visibles.

Ainsi, le procédé de détection d'une contamination à *Serpula lacrymans* dans un environnement intérieur selon

**EP 3 212 802 B1**

l'invention comprend les étapes suivantes :

a. le prélèvement d'un échantillon de composés organiques volatils (COV) dans l'environnement intérieur,
b. la détection de la présence ou l'absence de COV prédéterminés, émis par *Serpula lacrymans,* lesdits COV prédéterminés comprenant au moins un COV de chacune des trois catégories de COV suivantes :

(1). les COV émis par *Serpula lacrymans* quelle que soit la souche de *Serpula lacrymans* et qui n'ont pas d'autres origines ;
(2). les COV émis par *Serpula lacrymans* quelle que soit la souche de *Serpula lacrymans,* mais qui peuvent avoir d'autres origines ;
(3). les COV émis seulement et spécifiquement par certaines souches de *Serpula lacrymans* et qui n'ont pas d'autres origines ;

c. la détermination d'une présence ou d'une absence de contamination à *Serpula lacrymans* en fonction respectivement de la présence et de l'absence desdits COV prédéterminés, en prenant en considération chacune des conditions (i), (ii) et (iii) suivantes :

(i). la présence de COV de catégorie (1) indique directement la présence d'une contamination à *Serpula lacrymans* tandis que l'absence de tels COV indique l'absence d'une contamination à *Serpula lacrymans* ; et
(ii). la présence de COV de catégorie (2) ne permet pas de conclure sur une contamination à *Serpula lacrymans* tandis que l'absence de tels COV indique l'absence d'une contamination à *Serpula lacrymans ;* et
(iii). la présence de COV de catégorie (3) indique la présence d'une contamination à *Serpula lacrymans* tandis que leur absence ne permet pas de conclure sur l'absence d'une contamination à *Serpula lacrymans,*

les COV de la catégorie (1) étant l'isocyanure de méthyle.

Par « environnement intérieur » on entend une pièce confinée à l'intérieur d'un bâtiment ou d'une cavité naturelle. L'environnement intérieur peut être aéré de façon continu (par exemple par une ventilation forcée) ou non continu. Des exemples d'environnements intérieurs peuvent être trouvés dans des bâtiments tels que les habitations, les musées, les églises, les caves, les monuments historiques, les bâtiments administratifs, les écoles et les hôpitaux, mais aussi dans des cavités naturelles telles que les grottes. Dans la présente demande, le terme *Serpula lacrymans* fait référence à toutes les souches de *Serpula lacrymans.* Par « souches de *Serpula lacrymans* » on entend les différentes variantes génétiques de l'espèce *Serpula lacrymans.*
Par « COV émis par *Serpula lacrymans* » on entend les COV issues du métabolisme de *Serpula lacrymans.*
Les COV pouvant avoir « d'autres origines » sont des COV non spécifiques au métabolisme de *Serpula lacrymans* et peuvent provenir par exemple des matériaux de constructions ou de sources biologiques telles que les animaux, les végétaux, les bactéries ou les champignons autres que *Serpula lacrymans,* notamment les ascomycètes.
L'étape a) de prélèvement de l'échantillon de COV peut être réalisée par toute technique bien connue de l'homme du métier. Il peut s'agir d'un prélèvement passif, par exemple par échantillonnage diffusif sur un adsorbant solide de type carbographe 4. De préférence, le prélèvement est un prélèvement actif réalisé, par exemple par une pompe forçant le passage de l'air ambiant sur un absorbant solide de type Tenax®.
L'étape b) comprend la détection, à partir de l'échantillon de COV prélevé à l'étape a), de la présence ou l'absence de certains COV prédéterminés, émis lors du développement de *Serpula lacrymans.* Lesdits COV prédéterminés sont choisis parmi les catégories (1), (2) et (3) suivantes :

(1) les COV émis par *Serpula lacrymans* quelle que soit la souche de *Serpula lacrymans* et qui n'ont pas d'autres origines ;
(2) les COV émis par *Serpula lacrymans* quelle que soit la souche de *Serpula lacrymans,* mais qui peuvent avoir d'autres origines ;
(3) les COV émis seulement et spécifiquement par certaines souches de *Serpula lacrymans* et qui n'ont pas d'autres origines.

De préférence, l'étape b) comprend la détection de plusieurs COV d'au moins une des catégories précitées. Plus préférentiellement l'étape b) comprend la détection de plusieurs COV d'au moins deux des catégories précitées, par exemple au moins deux COV de chacune des catégories (2) et (3). Encore plus préférentiellement l'étape b) comprend la détection de plusieurs COV de chacune des trois catégories précitées.
Les COV de la catégorie (1) comprennent notamment l'isocyanure de méthyle. Les COV de la catégorie (2) comprennent notamment le 2-méthylfurane, le 2-méthyl-3-butan-2-ol, le dimétyldisulfide, le furfural, le 4-heptèn-2-one, l'alpha-pinène, le benzoate de méthyle et l'alpha-cubebène. Les COV de la catégorie (3) comprennent notamment l'isobutyronitrile, le trichlorométhane, le thioacétate de méthyle, le 2,5-diméthylfurane, le 3-méthyl-1,3,5-hexatriène, le 2(5H)-furanone, le 1-(2-furanyl)-éthanone, le méthylcarbamate de 3-méthylphényle, le 1-méthoxy-3-méthylbutane, le 5-heptèn-2-one, le 4-méthyl-5-hexèn-2-ol, l'acétate de 3-méthyl-3-butèn-1-ol, l'alcool benzylique et le 3-iodo-1-propène.

Dans un mode de réalisation particulier, les COV de la catégorie (2) consistent en le 2-méthyl-3-butan-2-ol, le furfural, le 4-heptèn-2-one, le benzoate de méthyle et l'alpha-cubebène ; et les COV de la catégorie (3) consistent en l'isobutyronitrile, le trichlorométhane, le thioacétate de méthyle, le 2,5-diméthylfurane, le 3-méthyl-1,3,5-hexatriène, le 1-(2-furanyl)-éthanone, le méthylcarbamate de 3-méthylphényle, le 1-méthoxy-3-méthylbutane, le 5-heptèn-2-one, le 4-méthyl-5-hexèn-2-ol, l'acétate de 3-méthyl-3-butèn-1-ol, l'alcool benzylique et le 3-iodo-1-propène.

De préférence, la présence ou l'absence des COV prédéterminés sont détectés par chromatographie en phase gazeuse suivie de spectrométrie de masse (GC/MS).

L'étape c) comprend la détermination d'une présence ou d'une absence de contamination à *Serpula lacrymans* en fonction respectivement de la présence et de l'absence desdits COV prédéterminés, en prenant en considération chacune des conditions (i), (ii) et (iii) suivantes :

> (i). la présence de COV de catégorie (1) indique directement la présence d'une contamination à *Serpula lacrymans* tandis que l'absence de tels COV indique l'absence d'une contamination à *Serpula lacrymans* ; et
> (ii). la présence de COV de catégorie (2) ne permet pas de conclure sur une contamination à *Serpula lacrymans* tandis que l'absence de tels COV indique l'absence d'une contamination à *Serpula lacrymans* ; et
> (iii). la présence de COV de catégorie (3) indique la présence d'une contamination à *Serpula lacrymans* tandis que leur absence ne permet pas de conclure sur l'absence d'une contamination à *Serpula lacrymans.*

Contrairement aux procédés classiques utilisant la détection de COV, le procédé selon la présente invention prend en compte non seulement la présence des COV prédéterminés mais également l'absence de ceux-ci. Ainsi, le procédé selon l'invention permet de déterminer avec plus de certitude la présence ou l'absence d'une contamination à *Serpula lacrymans.*

La détermination d'une présence ou d'une absence de contamination à *Serpula lacrymans* peut se faire avantageusement par le calcul d'un indice de contamination qui est basé sur la classification des COV prédéterminés dans les groupes (1) (2) et (3), et sur l'indication de la présence ou de l'absence de chacun des COV prédéterminés sur la présence ou l'absence d'une contamination à *Serpula lacrymans.* Ainsi, l'étape c) du procédé selon l'invention comprend de préférence :

> C1) l'attribution d'une valeur à chacun des COV prédéterminés en fonction de la présence ou l'absence dudit COV prédéterminé, en prenant en considération les conditions (i), (ii) et (iii), et
> C2) le calcul d'un indice de contamination à *Serpula lacrymans* correspondant à la somme de ces valeurs,

la présence d'une contamination à *Serpula lacrymans* étant détectée lorsque l'indice de contamination est supérieur à une valeur seuil prédéterminée.

Typiquement, l'attribution des valeurs à l'étape c1) est réalisée selon une échelle de valeur V1, V2 et V3, dans laquelle :

> V1 correspond à une indication sur la présence d'une contamination à *Serpula lacrymans ;*
> V2 correspond à une impossibilité de conclure sur une contamination à *Serpula lacrymans* ; et
> V3 correspond à une indication sur l'absence d'une contamination à *Serpula lacrymans.*

Ainsi, la présence d'un COV est incrémentée d'une valeur « V1 » si la présence du COV indique la présence d'une contamination fongique et d'une valeur « V2 » si la présence du COV ne permet pas de conclure à la présence d'une contamination fongique. L'absence d'un COV est incrémentée d'une valeur « V3 » si l'absence du COV indique l'absence d'une contamination fongique et d'une valeur « V2 » si l'absence du COV ne permet pas de conclure à l'absence d'une contamination fongique. Le tableau 1 ci-dessous résume le principe d'attribution des valeurs aux COV prédétermines selon une échelle de valeurs V1, V2 et V3.

**Tableau 1**

| Catégorie à laquelle appartient le COV prédéterminé | Valeur attribuée | |
|---|---|---|
| | présence | absence |
| Catégorie (1) | V1 | V3 |

(suite)

| Catégorie à laquelle appartient le COV prédéterminé | Valeur attribuée | |
|---|---|---|
| | présence | absence |
| Catégorie (2) | V2 | V3 |
| Catégorie (3) | V1 | V2 |

De préférence, V1, V2 et V3 satisfont la relation :

$$V1 > V2 > V3$$

Plus préférentiellement, l'intervalle entre les valeurs V1 et V2 est égal à l'intervalle entre les valeurs V2 et V3.
Dans un mode de réalisation particulier, V1 = -V3 et V2 = 0.

L'indice de contamination est calculé par addition des valeurs qui ont été attribuées à chacun des COV prédéterminés en fonction de leur présence ou de leur l'absence. Le résultat de cette addition, c'est-à-dire l'indice de contamination, indique s'il y a présence ou absence de contamination à *Serpula lacrymans*.

La conclusion d'une présence ou d'une absence d'une contamination à *Serpula lacrymans* en fonction de la valeur de l'indice de contamination dépend des valeurs données à V1, V2 et V3. Par exemple, lorsque V1, V2 et V3 satisfont la relation V1 > V2 > V3, une valeur élevée indique la présence d'une contamination de *Serpula lacrymans,* a contrario, une valeur faible l'exclut.

Plus particulièrement, un indice de contamination supérieur à une valeur seuil prédéterminée indique une présence de contamination à *Serpula lacrymans.* Au contraire, un indice de contamination inférieur ou égal à cette valeur seuil prédéterminée indique une absence de contamination à *Serpula lacrymans*.

La valeur seuil prédéterminée est fixée en fonction des valeurs V1, V2 et V3. Par exemple, lorsque le l'intervalle entre les valeurs V1 et V2 est égale à l'intervalle entre les valeurs V2 et V3, la valeur seuil prédéterminé pour l'indice de contamination est de préférence V3 multiplié par le nombre de COV prédéterminé détectés.

Dans un mode de réalisation préféré, V1 = +1, V2 = 0 et V3 = -1 et l'attribution des valeurs se fait de la façon suivante :

- la présence d'un COV de la catégorie (1) est caractérisée par la valeur +1 et son absence par la valeur -1 ;
- la présence d'un COV de la catégorie (2) est caractérisée par la valeur 0 et son absence par la valeur -1 ;
- la présence d'un COV de la catégorie (3) est caractérisée par la valeur +1 et son absence par la valeur 0.

Ainsi, l'indice de contamination est soit une valeur négative, soit égal à zéro, soit une valeur positive. La valeur seuil de l'indice de contamination est alors 0. Par conséquent, un indice de contamination inférieur ou égal à 0 indique l'absence d'une contamination à *Serpula lacrymans.* A contrario, un indice de contamination strictement positif indique la présence d'une contamination à *Serpula lacrymans.*

Le procédé de détection d'une contamination à *Serpula lacrymans* selon l'invention est particulièrement utile pour la détection précoce d'une telle contamination, c'est-à-dire avant l'apparition de signes visibles de contamination. Cette possibilité de détection précoce est de grande importance car des dommages conséquents ont en général déjà été causés quand les premiers signes visibles d'une contamination à *Serpula lacrymans* apparaissent.

L'exemple de réalisation suivant illustre la présente invention, sans en limiter en aucune façon la portée.

EXEMPLE :

Des prélèvements de COV *in situ* ont été réalisés par échantillonnage actif sur un adsorbant solide de type Tenax® dans différents environnements intérieurs constitués de dix-sept salles de sites patrimoniaux. Le prélèvement est assuré par une pompe. L'échantillonneur est composé d'une cartouche et d'une pompe. La cartouche cylindrique consiste en un tube en acier inoxydable de 90 mm de longueur et 5 mm de diamètre interne contenant un adsorbant solide (TENAX TA, 200 mg par tube) . Le prélèvement est réalisé sur site pendant 1 heure à 150 mL/min. Le point de prélèvement se situe entre 0,5 et 1 m de hauteur. La majeur partie des COV composant l'air de la salle sont alors piégés dans l'adsorbant.

Les tubes contenant l'adsorbant sont transférés dans une chaîne analytique du laboratoire. Cette chaîne consiste en la combinaison de deux techniques :

- la chromatographie en phase gazeuse (GC) utilisée pour séparer les COV,
- la spectrométrie de masse (MS) employée pour identifier ces composés.

Pour chacune des dix-sept salles, on obtient donc des chromatogrammes et des COV prédéterminés émis par *Serpula lacrymans* y sont recherchés. Les COV prédéterminés recherchés comprennent 1 COV de la catégorie (1), 8 COV de la catégorie (2) et 15 COV de la catégorie (3) (voir tableau 2 pour les COV prédéterminés et leur catégorie correspondante).

Un indice de contamination est ensuite calculé afin de regrouper l'ensemble des informations fournies par la présence ou l'absence des COV prédéterminés identifiés. Cet indice de contamination est calculé en attribuant la valeur +1, 0 ou -1 à chacun des COV prédéterminés de la façon suivante :

- la présence d'un COV de la catégorie (1) est caractérisée par la valeur +1 et son absence par la valeur -1 ;
- la présence d'un COV de la catégorie (2) est caractérisée par la valeur 0 et son absence par la valeur -1 ;
- la présence d'un COV de la catégorie (3) est caractérisée par la valeur +1 et son absence par la valeur 0.

D'après la construction de cet indice, une valeur positive rend probable la présence d'un développement de *Serpula lacrymans* dans la salle étudiée, a contrario, une valeur négative ou nulle l'exclut.

Le calcul de l'indice de contamination pour chacune des salles 1 à 17 est présenté dans le tableau 2.

TABLEAU 2 :

| COV prédéterminés (catégorie) | | Salle | | | | | | | | | | | | | | | | |
|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 | 17 |
| isocyanure d'éthyle | (1) | -1 | -1 | -1 | -1 | 1 | -1 | -1 | -1 | -1 | 1 | -1 | -1 | -1 | -1 | 1 | -1 | -1 |
| 2-méthylefurane | (2) | 0 | -1 | -1 | -1 | 0 | 0 | -1 | 0 | 0 | 0 | 0 | 0 | 0 | 0 | 0 | 0 | 0 |
| 2-méthyl-3-butèn-2-ol | (2) | -1 | -1 | -1 | -1 | -1 | -1 | -1 | 0 | -1 | 0 | 0 | 0 | 0 | -1 | 0 | 0 | -1 |
| diméthylsulfide | (2) | -1 | 0 | 0 | -1 | 0 | -1 | -1 | -1 | -1 | 0 | -1 | -1 | -1 | 0 | 0 | -1 | -1 |
| furfural | (2) | 0 | -1 | 0 | -1 | 0 | 0 | 0 | 0 | 0 | 0 | 0 | 0 | 0 | 0 | 0 | -1 | 0 |
| 4-heptèn-2-one | (2) | 0 | -1 | 0 | -1 | -1 | -1 | -1 | -1 | -1 | 0 | 0 | 0 | -1 | -1 | 0 | -1 | 0 |
| alpha-pinène | (2) | 0 | 0 | 0 | -1 | -1 | 0 | 0 | 0 | 0 | 0 | 0 | 0 | 0 | 0 | 0 | -1 | 0 |
| benzoate de méthyle | (2) | 0 | 0 | -1 | 0 | 0 | -1 | 0 | -1 | -1 | 0 | 0 | 0 | -1 | 0 | 0 | -1 | 0 |
| alpha-cubebène | (2) | 0 | -1 | 0 | -1 | -1 | 0 | -1 | 0 | 0 | 0 | 0 | 0 | -1 | -1 | 0 | -1 | 0 |
| isobutyronitrile | (3) | 0 | 0 | 0 | 0 | 0 | 0 | 0 | 0 | 0 | 0 | 0 | 0 | 0 | 0 | 0 | 0 | 0 |
| trichlorométhane | (3) | 0 | 1 | 0 | 0 | 0 | 0 | 0 | 1 | 1 | 0 | 1 | 1 | 1 | 1 | 1 | 0 | 1 |
| thioacétate de méthyle | (3) | 0 | 0 | 0 | 0 | 0 | 0 | 1 | 0 | 0 | 0 | 0 | 0 | 0 | 1 | 0 | 0 |
| 2,5-diméthylfurane | (3) | 0 | 0 | 0 | 0 | 0 | 0 | 0 | 0 | 0 | 0 | 1 | 0 | 0 | 0 | 0 | 0 | 0 |
| 3-méthyl-1,3,5-hexatriène | (3) | 0 | 0 | 0 | 0 | 0 | 0 | 0 | 1 | 0 | 0 | 0 | 0 | 0 | 1 | 1 | 1 |
| 2(5H)-furanone | (3) | 1 | 1 | 0 | 1 | 1 | 0 | 0 | 0 | 0 | 1 | 1 | 1 | 1 | 0 | 1 | 0 | 0 |
| 1-(2-furanyl)-éthanone | (3) | 0 | 0 | 1 | 0 | 0 | 0 | 0 | 0 | 0 | 0 | 0 | 0 | 0 | 1 | 0 | 0 |
| méthylcarbamate de 3-méthylphényle | (3) | 0 | 0 | 1 | 0 | 0 | 1 | 0 | 0 | 0 | 0 | 1 | 1 | 0 | 1 | 0 | 0 |
| 1-méthoxy-3-méthylbutane | (3) | 0 | 0 | 0 | 0 | 0 | 0 | 0 | 0 | 0 | 0 | 1 | 0 | 0 | 0 | 0 | 0 | 0 |
| 5-heptèn-2-one | (3) | 0 | 0 | 0 | 0 | 0 | 0 | 0 | 0 | 0 | 1 | 0 | 0 | 0 | 0 | 0 | 0 | 0 |
| 4-méthyl-5-hexèn-2-ol | (3) | 0 | 0 | 0 | 0 | 0 | 0 | 0 | 0 | 0 | 0 | 0 | 0 | 0 | 0 | 0 | 0 | 0 |
| l'acétate de 3-méthyl-3-butèn-1-ol | (3) | 0 | 0 | 0 | 0 | 0 | 0 | 0 | 0 | 0 | 0 | 0 | 0 | 0 | 0 | 0 | 0 | 0 |
| l'alcool benzylique | (3) | 0 | 0 | 0 | 0 | 0 | 0 | 0 | 0 | 0 | 0 | 0 | 0 | 0 | 0 | 0 | 0 | 0 |
| 3-iodo-1-propène | (3) | 0 | 0 | 0 | 0 | 0 | 0 | 0 | 0 | 0 | 0 | 0 | 0 | 0 | 0 | 0 | 0 | 0 |
| **Indice de contamination** | | -2 | -4 | -2 | -7 | -2 | -4 | -5 | -3 | -3 | 3 | 1 | 3 | -3 | -3 | 7 | -6 | -1 |

EP 3 212 802 B1

L'interprétation de l'indice de contamination selon l'invention suggère que seule les salles 10, 11, 12 et 15, pour lesquelles l'indice de contamination est positif, présentent une contamination à *Serpula lacrymans.*

Les dix-sept salles ont été investigués indépendamment afin de déterminer leur état réel de contamination à *Serpula lacrymans* et à d'autres champignons. Les dix-sept salles ont pu être classées en quatre groupes en fonction de leur état de contamination réel :

- huit salles (salles 1 à 5, 7, 8 et 14) ne présentaient aucun développement fongique (ni mérule, ni ascomycètes) ;
- quatre salles (salles 10 à 12 et 15) présentaient un développement à la mérule ;
- deux salles (salles 6 et 17) présentaient un développement d'ascomycètes sans développement de mérule ; et
- trois salles (salles 9, 13 et 16) avaient été réhabilitées après une contamination à la mérule par un traitement chimique et/ou physique.

L'ensemble des cas de contamination à *Serpula lacrymans* dans les environnements testés a été détecté avec l'indice de contamination selon l'invention, alors que les environnements non contaminés à *Serpula lacrymans* ont induit un score d'indice négatif. Ainsi l'indice de contamination selon l'invention n'a conduit à aucun faux-négatifs ou faux-positifs.
De plus, les environnements contaminés par des ascomycètes (microorganismes pourtant proche de *Serpula lacrymans*) mais exempts de développement de *Serpula lacrymans* restent négatifs. Cette observation montre la spécificité de l'indice de contamination selon l'invention. Cette spécificité, essentielle pour limiter les cas de faux-positifs, est d'autant plus importante que la présence de *Serpula lacrymans* s'accompagne fréquemment de développements d'ascomycètes alors que la réciproque est fausse. Enfin, les environnements réhabilités présentent des valeurs d'indice de contamination négatives, montrant d'une part l'absence de composés résiduels, et d'autre part l'absence d'interférence avec les traitements appliqués. Par conséquent, l'indice de contamination selon l'invention peut également être utilisé pour le contrôle de remédiations d'environnements anciennement contaminés à *Serpula lacrymans*.

## Revendications

1. Procédé de détection d'une contamination à *Serpula lacrymans* dans un environnement intérieur comprenant les étapes suivantes :

   a. le prélèvement d'un échantillon de composés organiques volatils (COV) dans l'environnement intérieur,
   b. la détection de la présence ou l'absence de COV prédéterminés, émis par *Serpula lacrymans,* lesdits COV prédéterminés comprenant au moins un COV de chacune des trois catégories de COV suivantes :

   (1). les COV émis par *Serpula lacrymans* quelle que soit la souche de *Serpula lacrymans* et qui n'ont pas d'autres origines ;
   (2). les COV émis par *Serpula lacrymans* quelle que soit la souche de *Serpula lacrymans,* mais qui peuvent avoir d'autres origines ;
   (3). les COV émis seulement et spécifiquement par certaines souches de *Serpula lacrymans* et qui n'ont pas d'autres origines ;

   c. la détermination d'une présence ou d'une absence de contamination à *Serpula lacrymans* en fonction respectivement de la présence et de l'absence desdits COV prédéterminés, en prenant en considération chacune des conditions (i), (ii) et (iii) suivantes :

   (i). la présence de COV de catégorie (1) indique directement la présence d'une contamination à *Serpula lacrymans* tandis que l'absence de tels COV indique l'absence d'une contamination à *Serpula lacrymans* ; et
   (ii). la présence de COV de catégorie (2) ne permet pas de conclure sur une contamination à *Serpula lacrymans* tandis que l'absence de tels COV indique l'absence d'une contamination à *Serpula lacrymans* ; et
   (iii). la présence de COV de catégorie (3) indique la présence d'une contamination à *Serpula lacrymans* tandis que leur absence ne permet pas de conclure sur l'absence d'une contamination à *Serpula lacrymans,*

   ledit procédé étant **caractérisé en ce que**
   les COV de la catégorie (1) sont l'isocyanure de méthyle.

2. Procédé selon la revendication 1, **caractérisé en ce que**

- les COV de la catégorie (2) sont choisis dans le groupe consistant en le 2-méthylfurane, le 2-méthyl-3-butan-2-ol, le dimétyldisulfide, le furfural, le 4-heptèn-2-one, l'alpha-pinène, le benzoate de méthyle et l'alpha-cubebène,
- les COV de la catégorie (3) sont choisis dans le groupe consistant en l'isobutyronitrile, le trichlorométhane, le thioacétate de méthyle, le 2,5-diméthylfurane, le 3-méthyl-1,3,5-hexatriène, le 2(5H)-furanone, le 1-(2-furanyl)-éthanone, le méthylcarbamate de 3-méthylphényle, le 1-méthoxy-3-méthylbutane, le 5-heptèn-2-one, le 4-méthyl-5-hexèn-2-ol, l'acétate de 3-méthyl-3-butèn-1-ol, l'alcool benzylique et le 3-iodo-1-propène.

**3.** Procédé selon la revendication 1 ou 2, **caractérisé en ce que** l'étape c) comprend

C1) l'attribution d'une valeur à chacun des COV prédéterminés en fonction de la présence ou l'absence dudit COV prédéterminé, en prenant en considération les conditions (i), (ii) et (iii), et
C2) le calcul d'un indice de contamination à *Serpula lacrymans* correspondant à la somme de ces valeurs,

la présence d'une contamination à *Serpula lacrymans* étant détectée lorsque l'indice de contamination est supérieur à une valeur seuil prédéterminée.

**4.** Procédé selon la revendication 3, **caractérisé en ce que** dans l'étape c1) l'attribution des valeurs est réalisée selon une échelle de valeurs V1, V2 et V3, dans laquelle :

V1 correspond à une indication sur la présence d'une contamination à *Serpula lacrymans* ;
V2 correspond à une impossibilité de conclure sur une contamination à *Serpula lacrymans* ; et
V3 correspond à une indication sur l'absence d'une contamination à *Serpula lacrymans*.

**5.** Procédé selon la revendication 4, **caractérisé en ce que** V1 = +1, V2 = 0 et V3 = -1 et l'attribution des valeurs se fait de la façon suivante :

- la présence d'un COV de la catégorie (1) est **caractérisée par** la valeur +1 et son absence par la valeur -1 ;
- la présence d'un COV de la catégorie (2) est **caractérisée par** la valeur 0 et son absence par la valeur -1 ;
- la présence d'un COV de la catégorie (3) est **caractérisée par** la valeur +1 et son absence par la valeur 0 ;

un indice strictement positif signifiant une présence de contamination à *Serpula lacrymans* et un indice négatif ou nul signifiant une absence de contamination à *Serpula lacrymans*.

## Patentansprüche

**1.** Ein Verfahren zur Detektion einer *Serpula lacrymans* Kontamination in einem Innenraum, umfassend die folgenden Schritte:

a. die Entnahme einer Probe von flüchtigen organischen Verbindungen (VOC) im Innenraum,
b. die Detektion der Anwesenheit oder der Abwesenheit vorbestimmter VOC, die von *Serpula lacrymans* emittiert sind, wobei die vorbestimmten VOC mindestens eine VOC von jeder der drei folgenden VOC Kategorien umfassen:

(1). die von *Serpula lacrymans* emittierten VOC, unabhängig vom *Serpula lacrymans* Stamm, und die keinen anderen Ursprung haben;
(2). die von *Serpula lacrymans* emittierte VOC, unabhängig vom *Serpula lacrymans* Stamm, die jedoch andere Ursprünge haben können;
(3). die VOC, die nur und spezifisch von bestimmten *Serpula lacrymans* Stämmen emittiert werden und keine anderen Ursprünge haben;

c. die Bestimmung einer Anwesenheit oder einer Abwesenheit einer *Serpula lacrymans* Kontamination auf der Grundlage der Anwesenheit beziehungsweise der Abwesenheit der vorbestimmten VOC unter Berücksichtigung jeder der folgenden Bedingungen (i), (ii) und (iii):

(i). die Anwesenheit von VOC der Kategorie (1) zeigt direkt die Anwesenheit einer *Serpula lacrymans* Kontamination an, während die Abwesenheit derartiger VOC die Abwesenheit einer *Serpula lacrymans* Kontamination anzeigt; und

(ii). die Anwesenheit von VOC der Kategorie (2) lässt nicht auf eine Kontamination mit *Serpula lacrymans* schließen, während die Abwesenheit derartiger VOC die Abwesenheit einer *Serpula lacrymans* Kontamination anzeigt; und

(iii). die Anwesenheit von VOC der Kategorie (3) zeigt die Anwesenheit einer *Serpula lacrymans* Kontamination an, während ihre Abwesenheit keine Rückschlüsse auf die Abwesenheit einer *Serpula lacrymans* Kontamination zulässt,

wobei das Verfahren **dadurch gekennzeichnet ist, dass**
die VOC der Kategorie (1) Methylisocyanid sind.

2. Verfahren nach Anspruch 1, **dadurch gekennzeichnet, dass**

- die VOC der Kategorie (2) ausgewählt sind aus der Gruppe bestehend aus 2-Methylfuran, 2-Methyl-3-butan-2-ol, Dimethyldisulfid, Furfural, 4-Hepten-2-on, alpha-Pinen, Methylbenzoat und alpha-Cubeben,
- die VOC der Kategorie (3) ausgewählt sind aus der Gruppe bestehend aus Isobutyronitril, Trichlormethan, Methylthioacetat, 2,5-Dimethylfuran, 3-Methyl-1,3,5-hexatrien, 2(5H)-Furanon, 1-(2-Furanyl)ethanon, 3-Methylphenylmethylcarbamat, 1-Methoxy-3-methylbutan, 5-Hepten-2-on, 4-Methyl-5-hexen-2-ol, 3-Methyl-3-buten-1-ol-acetat, Benzylalkohol und 3-Iod-1-propen.

3. Verfahren nach Anspruch 1 oder 2, **dadurch gekennzeichnet, dass** Schritt c) umfasst

C1) das Zuweisen eines Werts zu jeder der vorbestimmten VOC gemäß der Anwesenheit oder der Abwesenheit der vorbestimmten VOC unter Berücksichtigung der Bedingungen (i), (ii) und (iii) und
(C2) die Berechnung eines *Serpula lacrymans* Kontaminationsindexes entsprechend der Summe dieser Werte,

wobei die Anwesenheit einer *Serpula lacrymans* Kontamination detektiert wird, wenn der Kontaminationsindex größer als ein vorbestimmter Schwellenwert ist.

4. Verfahren nach Anspruch 3, **dadurch gekennzeichnet, dass** in Schritt c1) das Zuweisen der Werte nach einer Werteskala V1, V2 und V3 realisiert wird, bei der:

V1 einem Hinweis auf die Anwesenheit einer *Serpula lacrymans* Kontamination entspricht;
V2 einer Unmöglichkeit auf eine *Serpula lacrymans* Kontamination zu schließen entspricht; und
V3 einem Hinweis auf die Abwesenheit einer *Serpula lacrymans* Kontamination entspricht.

5. Verfahren nach Anspruch 4, **dadurch gekennzeichnet, dass** V1 = +1, V2 = 0 und V3 = -1 und dass das Zuweisen der Werte wie folgt erfolgt:

- die Anwesenheit einer VOC der Kategorie (1) wird durch den Wert +1 und ihre Abwesenheit durch den Wert -1 charakterisiert;
- die Anwesenheit einer VOC der Kategorie (2) wird durch den Wert 0 und ihre Abwesenheit durch den Wert -1 charakterisiert;
- die Anwesenheit einer VOC der Kategorie (3) wird durch den Wert +1 und ihre Abwesenheit durch den Wert 0 charakterisiert;

wobei ein streng positiver Index eine Anwesenheit einer *Serpula lacrymans* Kontamination bedeutet und ein negativer oder Null-Index eine Abwesenheit einer *Serpula lacrymans* Kontamination bedeutet.

**Claims**

1. A method for detecting *Serpula lacrymans* contamination in an internal environment, comprising the following steps:

a. taking a sample of volatile organic compounds (VOCs) from the internal environment,
b. detecting the presence or absence of predetermined VOCs, emitted by *Serpula lacrymans,* said predetermined VOCs comprising at least one VOC from each of the following three VOC categories:

(1). VOCs emitted by *Serpula lacrymans* whatever the *Serpula lacrymans* strain and which do not have other origins;

(2). VOCs emitted by *Serpula lacrymans* whatever the *Serpula lacrymans* strain, but which may have other origins;

(3). VOCs emitted only and specifically by certain *Serpula lacrymans* strains and which do not have other origins;

c. determining a presence or an absence of *Serpula lacrymans* contamination as a function respectively of the presence and of the absence of said predetermined VOCs, taking into consideration each of the following conditions (i), (ii) and (iii):

(i). the presence of VOCs of category (1) directly indicates the presence of *Serpula lacrymans* contamination, while the absence of such VOCs indicates the absence of *Serpula lacrymans* contamination; and

(ii). the presence of VOCs of category (2) does not make it possible to reach a conclusion regarding *Serpula lacrymans* contamination, whereas the absence of such VOCs indicates the absence of *Serpula lacrymans* contamination; and

(iii). the presence of VOCs of category (3) indicates the presence of *Serpula lacrymans* contamination, whereas their absence does not make it possible to reach a conclusion regarding the absence of *Serpula lacrymans* contamination,

said method being **characterized in that**
the VOCs of category (1) consist of methyl isocyanide.

2. The method according to claim 1, **characterized in that**

- the VOCs of category (2) are chosen from the group consisting of 2-méthylfuran, 2-methyl-3-butan-2-ol, dimethyl disulfide, furfural, 4-hepten-2-one, alpha-pinene, methyl benzoate and alpha-cubebene,
- the VOCs of category (3) are chosen from the group consisting of isobutyronitrile, trichloromethane, methyl thioacetate, 2,5-dimethylfuran, 3-methyl-1,3,5-hexatriene, 2(5H)-furanone, 1-(2-furanyl)ethanone, 3-methylphenyl methylcarbamate, 1-methoxy-3-methylbutane, 5-hepten-2-one, 4-methyl-5-hexen-2-ol, 3-methyl-3-buten-1-ol acetate, benzyl alcohol and 3-iodo-1-propene.

3. The method according to claim 1 or 2, **characterized in that** step c) comprises

C1) assigning a value to each of the predetermined VOCs as a function of the presence or absence of said predetermined VOC, taking into consideration the conditions (i), (ii) and (iii), and
C2) calculating a *Serpula lacrymans* contamination index corresponding to the sum of these values,

the presence of *Serpula lacrymans* contamination being detected when the contamination index is above a predetermined threshold value.

4. The method according to claim 3, **characterized in that**, in step c1), the assigning of the values is carried out according to a scale of values V1, V2 and V3, in which:

V1 corresponds to an indication regarding the presence of *Serpula lacrymans* contamination;
V2 corresponds to it being impossible to reach a conclusion regarding *Serpula lacrymans* contamination; and
V3 corresponds to an indication regarding the absence of *Serpula lacrymans* contamination.

5. The method according to claim 4, **characterized in that** V1 = +1, V2 = 0 and V3 = -1 and the assigning of the values is carried out in the following way:

- the presence of a VOC of category (1) is **characterized by** the value +1 and its absence by the value -1;
- the presence of a VOC of category (2) is **characterized by** the value 0 and its absence by the value -1;
- the presence of a VOC of category (3) is **characterized by** the value +1 and its absence by the value 0;

a strictly positive index signifying that *Serpula lacrymans* contamination is present and a negative or zero index signifying that *Serpula lacrymans* contamination is absent.

## RÉFÉRENCES CITÉES DANS LA DESCRIPTION

**Documents brevets cités dans la description**

- FR 2913501